# EUROPÄISCHE PATENTSCHRIFT

(11) **EP 0 955 020 B1**
(45) Veröffentlichungstag und Bekanntmachung des Hinweises auf die Patenterteilung: **10.03.2004**
(21) Anmeldenummer: 99108312.2
(22) Anmeldetag: 28.04.1999
(51) Int. Cl.: A61F 2/38

(54) **Kniegelenk-Endoprothesen-Bausatz**
Assembly set for knee prostheses
Eléments d' assemblage pour endoprothèses du genou

(30) Priorität: 29.04.1998 DE 19819180
(43) Veröffentlichungstag der Anmeldung: 10.11.1999
(73) Patentinhaber: Muno, Dietmar, 66299 Friedrichsthal (DE)
(72) Erfinder: Muno, Dietmar, 66299 Friedrichsthal (DE)
(74) Vertreter: Patentanwälte Hauck, Graalfs, Wehnert, Döring, Siemons, Schildberg

(56) Entgegenhaltungen:
- EP-A- 0 519 873
- WO-A-96/24311
- FR-A- 2 735 017
- US-A- 5 282 868

## Beschreibung

Die vorliegende Erfindung betrifft einen Kniegelenk-Endoprothesen-Bausatz.

Es sind diverse Kniegelenk-Endoprothesen bekannt. Eine relativ alte Lösung hierfür ist in der DE 26 60 623 C 2 beschrieben. Diese Lösung umfasst ein Femurteil, ein Tibiateil sowie ein Zwischenteil, das als Lagerplatte ausgebildet ist, die auf Ihrer Oberseite konkave Gleitflächen aufweist, auf denen komplementär von ausgebildete Gleitflächen des Femurteiles abgestützt sind, und auf ihrer Unterseite eine ebene Gleitfläche besitzt, die auf der Lagerfläche des Tibiateiles abgestützt und um eine parallel zum Tibiaknochen verlaufende ideelle Achse drehbar ist. Dieses Zwischenteil oder Rotationsplateau wird dabei zur drehbaren Lagerung von Seitenwänden des Tibiateiles geführt, die die gleiche Krümmung besitzen wie die Aussenfläche des Rotationsplateaus.

Eine andere Lösung ist aus der EP 0 678 286 A 1 bekannt. Bei dieser Lösung erstreckt sich von einer Plattform einer Tibiakomponente ein ringförmiger Flansch nach oben, der zur Lagerung eines Rotationsplateaus dient, welches die Femurkomponente abstützt. Bei einer weiteren bekannten Lösung werden anstelle eines einstückigen Rotationsplateaus als Zwischenteil zur Abstützung der Femurkomponente getrennte Gleitlager verwendet, die geführt in einer Schienenkonstruktion laufen, welche von einem zentralen Ansatz und Seitenwänden einer Tibiaplattform gebildet wird. Diese beiden getrennten Gleitlager können somit eine geführte Anterior-Posterior-Bewegung durchführen.

Weitere Veröffentlichungen, in denen entsprechende Lösungen des Standes der Technik beschrieben sind, sind beispielsweise die US-PS en 4 309 778, 4 340 978, 4 219 893 und 4 085 466 sowie die EP-A-0 018 364 und US 5 282 868.

Die in den vorstehend genannten Veröffentlichungen beschriebenen Lösungen verfolgen alle das Ziel, trotz kongruentem Flächenkontakt der Gleitpartner eine entsprechende freie Beweglichkeit in der Prothesenkonstruktion zu erhalten. Dabei kann man grundlegend zwischen zwei Lösungsmöglichkeiten unterscheiden, nämlich einer, bei dem das die Femurkomponente abstützende Teil eine Rotationsbewegung ausführen kann, und einer, bei dem getrennte Gleitlager für die Femurkomponente vorgesehen sind, die eine geführte Anterior-Posterior-Bewegung durchführen können.

Welche der vorstehend beschriebenen Lösungen des Standes der Technik verwendet wird, hängt im wesentlichen vom Vorhandensein oder der Beschaffenheit des hinteren Kreuzbandes ab. Ist beispielsweise ein hinteres Kreuzband zum Zeitpunkt der Implantation noch funktionsfähig erhalten, so wird aufgrund der physiologischen Rückverlagerung des Femurs auf der Tibia eine prothetische Lösung angestrebt, bei der eine Imitation der Physiologie gegeben ist, d.h. die Gleitlager auch nach hinten gleiten können, wie dies bei der vorstehend beschriebenen Ausführungsform des Standes der Technik mit der Anordnung von zwei getrennten geführten Gleitlagern der Fall ist. Für den Fall, daß das Kreuzband nicht mehr funktionsfähig oder gar nicht mehr vorhanden ist, werden Lösungen eingesetzt, bei denen das Gleitlager (Rotationsplateau) nur ausschließlich rotieren kann. Diese Lösungen können daher keine Rückverlagerung des Femurs auf der Tibia imitieren und haben somit bei intaktem hinteren Kreuzband einen primär funktionellen Nachteil.

Beide Lösungsarten des Standes der Technik sind daher für einen speziellen Anwendungsfall konzipiert, der vom Vorhandensein bzw. der Funktionsfähigkeit des hinteren Kreuzbandes abhängig ist. Ein Einsatz für den anderen Anwendungsfall ist mit entsprechenden Nachteilen verbunden, nämlich einerseits einer unzureichenden Stabilität des Gelenkes, sofern die Variante mit den einzelnen, beweglichen Gleitlagern gewählt wurde, und andererseits einer mangelnden Bewegungsmöglichkeit bei der Variante mit dem um eine feste Achse drehbaren Rotationsplateau.

Aufgabe der vorliegenden Erfindung ist es, ein modulares Prothesensystem zu schaffen, das es unter Zugrundelegung der vorstehend beschriebenen Lösungen zuläßt, intraoperativ von der einen auf die andere Lösung umsteigen zu können, ohne die Tibiakomponente, d.h. das Trägerteil für das Zwischenteil (Gleitlager, Rotationsplateau), entfernen zu müssen.

Diese Aufgabe wird erfindungsgemäß durch einen Kniegelenk-Endoprothesen-Bausatz gelöst, der die folgenden Bestandteile umfaßt:
(a) Eine Trägerplattform mit einer Lagerfläche und einer nach oben vorstehenden medialen sowie einer nach oben vorstehenden lateralen Seitenwand, die sich gegenüberliegen und Teile einer Zylinderwand mit definiertem Radius bilden, so wie einem sich nach unten erstreckenden zentralen Stiel, in dem eine zentrale Ausnehmung angeordnet ist;
(b) einen Aufsatz zur zentralen drehfesten Anordnung auf der Trägerplattform;
(c) zwei zur Lagerung einer Femurkomponente dienende Gleitlager zur beweglichen Installation zwischen dem Aufsatz und den Seitenwänden der Trägerplattform; und
(d) ein zur Lagerung einer Femurkomponente dienendes Rotationsplateau zur zentralen drehbaren Installation auf der Trägerplattform mit Hilfe eines in die Ausnehmung der Trägerplattform eingreifenden Zapfens als Ersatz für den Aufsatz und die Gleitlager.

Die erfindungsgemäße Lösung geht von der Problematik aus, daß ein zum Operationszeitpunkt ursprünglich intaktes hinteres Kreuzband nach mehreren Jahren seine Funktionsfähigkeit verlieren kann. Die Folge davon ist ein instabiles Gelenk, sofern die vorstehend beschriebene Variante mit den einzelnen, beweglichen Gleitlagern implantiert wurde. Zu diesem Zeitpunkt müsste nunmehr eine Variante mit einem ausschließlich rotationsfähigen Gleitlager gewählt werden. Dies ist jedoch nur möglich, wenn alle bereits implantierten Prothesenteile operativ getauscht werden. Die erfindungsgemäße Lösung schafft hierfür Abhilfe, da sie es ermöglicht, intraoperativ von der einen auf die andere Lösung umsteigen zu können, ohne die bereits implantierte Tibiakomponente (Trägerplattform) entfernen zu müssen.

Natürlich schließt die erfindungsgemäße Lösung nicht aus, daß der Operateur, beispielsweise beim vollständigen Fehlen des hinteren Kreuzbandes, direkt ein zur Lagerung einer Femurkomponente dienendes Rotationsplateau implantiert, das ausschließich eine Rotationsbewegung durchführen kann. In jedem Falle bietet der Bausatz dem Operateur die Möglichkeit, je nach Anwendungsfall (in Abhängigkeit vom Vorhandensein oder Fehlen des hinteren Kreuzbandes) eine Variante mit getrennten beweglichen Gleitlagern oder eine Variante mit Rotationsplateau zu implantieren, wobei er die gleiche Tibiakomponente, d.h. das gleiche Trägerelement (Trägerplattform) verwenden kann. Der erfindungsgemäße Bausatz kommt bevorzugt dann zum Einsatz, wenn bei noch vorhandenem hinteren Kreuzband anfangs getrennte Gleitlager implantiert werden, die intraoperativ später bei Schwächung des hinteren Kreuzbandes durch ein Rotationsplateau ersetzt werden.

Zur Implantation der getrennten beweglichen Gleitlager sieht die Erfindung einen Aufsatz zur zentralen drehfesten Anordnung auf der Trägerplattform vor, der die innere Führung für die Gleitlager bildet. Wenn die Gleitlager später durch ein Rotationsplateau ersetzt werden sollen, wird dieser Aufsatz entfernt, und es wird stattdessen das Rotationsplateau mit Hilfe eines in die zentrale Ausnehmung der Trägerplattform eingreifenden Zapfens drehbar installiert. Ein Ersatz der Trägerplattform ist hierbei nicht erforderlich.

Vorzugsweise besitzen die Seitenwände der Trägerplattform und die äußere seitliche Begrenzungsfläche des Aufsatzes die gleiche Krümmung. Zwischen den Seitenwänden und dem Aufsatz entstehen hierbei Bereiche, die Teilen eines Kreisringes entsprechen und zur Aufnahme von je einem Gleitlager dienen, welche sich entlang diesen Teilkreisringbereichen geführt nach vorne und nach hinten bewegen können. Eine Festlegung der Gleitlager in axialer Richtung kann dabei über einen schwalbenschwanzförmigen Eingriff zwischen den Gleitlagern und dem zentralen Aufsatz erfolgen. Natürlich können sich die Gleitlager auch über dem zentralen Aufsatz zur Gelenkachse hin erstrecken.

Die Oberflächen der Gleitlager sind entsprechend konkav ausgebildet, um eine geeignete Femurkomponente abzustützen. Diese Ausgestaltung ist bekannt und ist nicht Gegenstand der vorliegenden Erfindung.

Auch die äußeren seitlichen Begrenzungsflächen des Rotationsplateaus und die Seitenwände der Trägerplattform besitzen vorzugsweise die gleiche Krümmung, wobei das Rotationsplateau vorzugsweise an den Seitenwänden der Trägerplattform relativ hierzu drehbar anliegt. Bei dieser Ausführungsform bilden daher die Seitenwände der Trägerplattform Führungsflächen für das Rotationsplateau, die ausschließlich eine Rotationsbewegung des Plateaus zulassen. Besonders bevorzugt wird eine Lösung, bei der das Rotationsplateau sowohl an den Seitenflächen der Trägerplattform als auch mit seinem Zapfen in der Ausnehmung der Trägerplattform geführt ist. Diese Ausführungsform bietet im bezug auf vorhandene Toleranzen die höchstmögliche Sicherheit in bezug auf eine Durchführung einer exakten Rotationsbewegung des Rotationsplateaus.

Bei einer speziellen Ausführungsform der Erfindung sind Rotationsplateau und Zapfen einstückig ausgebildet. Diese Ausführungsform kommt dann zum Einsatz, wenn eine Implantation eines derartigen Rotationsplateaus ohne Probleme, insbesondere Platzprobleme, möglich ist. Dabei wird die Fixierung des Aufsatzes an der Trägerplatte gelöst, und der Aufsatz sowie die beiden Gleitlager werden von der Trägerplattform entfernt. Danach wird das Rotationsplateau durch Einführung des Zapfens in die Ausnehmung der Trägerplattform installiert. Ausnehmung und Zapfen sind vorzugsweise konisch ausgebildet.

Eine weitere Variante der erfindungsgemäßen Lösung zeichnet sich dadurch aus, daß Rotationsplateau und Zapfen zweiteilig ausgebildet und über Befestigungseinrichtungen aneinander befestigbar sind. Diese Lösung kommt beispielsweise dann zum Einsatz, wenn aus Platzgründen etc. eine einstückige Variante des Rotationsplateaus mit Zapfen nicht implantiert werden kann. Die Installation des Rotationsplateaus erfolgt hierbei insbesondere intraoperativ so, daß nach Abkopplung des Zentralaufsatzes das Rotationsplateau (ohne Zapfen) von schräg anterior zwischen die Seitenwände der Trägerplattform eingesetzt wird. Zur Stabilisierung wird nun von oben durch eine zentrale Öffnung des Rotationsplateaus ein Drehzapfen in Richtung distal in die zentrale Ausnehmung der Trägerplattform eingesetzt und mit der Rotationsplattform (Rotationsgleitlager) fest verbunden. Ein Austausch der Gleitlager, wie vorstehend beschrieben, wäre ohne ein zweiteiliges Rotationsplateau in sehr vielen Fällen nicht möglich. Sollte es allerdings möglich sein, kann das zweiteilige Rotationsplateau auch ausserhalb des Körpers montiert und im vormontierten Zustand eingesetzt werden.

Die erfindungsgemäße Lösung umfasst ferner zweckmässigerweise eine Begrenzungseinrichtung für die Drehung des Rotationsplateaus, um beispielsweise dessen Drehung bis auf 25° zu begrenzen. Die Begrenzungseinrichtung wird vorzugsweise durch eine Nase an der Trägerplattform und eine hiermit zusammenwirkende Ausnehmung am Rotationsplateau gebildet.

Diese Nase findet vorzugsweise auch Anwendung zur Fixierung des Aufsatzes an der Trägerplattform. Bei dieser Lösung besitzt der Aufsatz eine mit der Nase der Trägerplattform zusammenwirkende Ausnehmung und ist über ein sich von der Nase in den Aufsatz erstreckendes Fixierelement, beispielsweise eine Fixierschraube, an der Trägerplattform fixiert. Auch dies verdeutlicht, daß die Trägerplattform nicht ausgetauscht werden muss und sowohl zur Aufnahme des Aufsatzes als auch zur Aufnahme der Rotationsplattform dient.

In Weiterbildung der Erfindung besitzt das Rotationsplateau eine nach oben ragende Stabilisierungsnase zur Aufnahme eines Femurteiles, insbesondere eines speziellen Femurteiles mit Stabilisierungskasten mit oder ohne Kopplung. Bei dieser Variante ist das Rotationsplateau vorzugsweise einstückig mit Zapfen ausgebildet.

Am unteren Ende des Stieles der Trägerplattform sind vorzugsweise Befestigungsmittel für Verlängerungsstiele vorgesehen. Bei diesen Befestigungsmitteln kann es sich beispielsweise um ein Schraubgewinde handeln.

Trägerplattform und Aufsatz bestehen vorzugsweise aus Metall, während Gleitlager und Rotationsplateau zweckmässigerweise aus Kunststoff bestehen. Als Kunststoff findet vorzugsweise Polyethylen Verwendung. Eine Ausbildung von Trägerplattform und Aufsatz aus Keramik wird ebenfalls bevorzugt.

Die Erfindung wird nachfolgend anhand eines Ausführungsbeispiels in Verbindung mit der Zeichnung im einzelnen erläutert. Es zeigen:
- Figur 1: eine Draufsicht auf eine Ausführungsform einer Kniegelenk-Endoprothese mit einer Trägerplattform, einem Aufsatz und zwei beweglichen Gleitlagern;
- Figur 2: einen Vertikalschnitt entlang Linie I-I in Figur 1 durch die Prothese der Figur 1;
- Figur 3: eine Draufsicht und einen Vertikalschnitt im umgedrehten Zustand durch den in den Figuren 1 und 2 gezeigten Aufsatz;
- Figur 4: einen Vertikalschnitt entlang Linie II-II in Figur 6 durch ein Rotationsplateau;
- Figur 5: eine Draufsicht und einen Vertikalschnitt entlang Linie III-III in Figur 5 einer Trägerplattform, die in Verbindung mit dem in Figur 4 gezeigten Rotationsplateau verwendet wird;
- Figur 6: eine Draufsicht auf das in Figur 4 gezeigte Rotationsplateau;
- Figur 7: eine Unteransicht des Rotationsplateaus der Figur 4;
- Figur 8: eine Seiten- und Schnittansicht entlang Linie II-II in Figur 6 des Rotationsplateaus;
- Figur 9: eine Draufsicht auf die Trägerplattform mit darauf installiertem, um 25° gedrehten Rotationsplateau;
- Figur 10: eine auseinandergezogene Ansicht der Teile eines zweiteiligen Rotationsplateaus mit Trägerplattform;
- Figur 11: einen Vertikalschnitt entlang Linie IV-IV in Figur 10 durch die Prothese im auf der Trägerplattform montierten Zustand des Rotationsplateaus; und
- Figur 12: eine weitere Ausführungsform einer Kniegelenk-Endoprothese, wobei Figur 12a eine Draufsicht auf die beiden beweglichen Gleitlager, Figur 12b einen Vertikalschnitt durch ein Gleitlager, Figur 12c eine Seitenansicht der zentralen Führung für die Gleitlager und Figur 12d eine Seitenansicht der zusammengebauten Prothese zeigen.

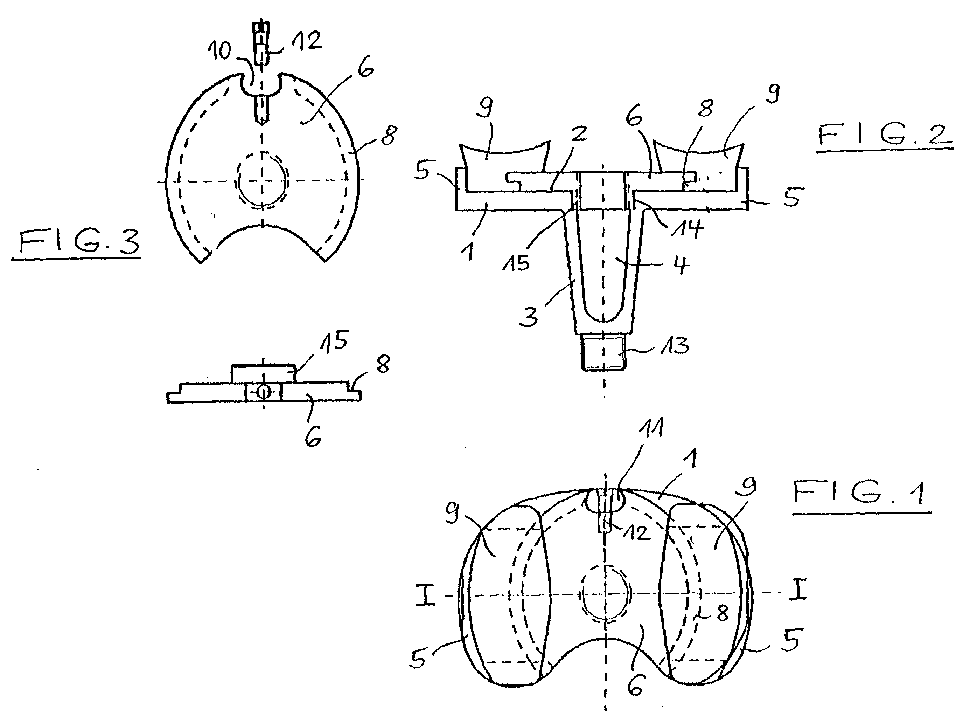

Es wird nunmehr eine Ausführungsform eines Kniegelenk-Endoprothesen-Bausatzes erläutert, der eine metallische Trägerplattform, einen metallischen Aufsatz, zwei Gleitlager aus Kunststoff und ein Rotationsplateau aus Kunststoff umfasst. Die Figuren 1 und 2 zeigen eine aus dem Bausatz hergestellte Prothese (ohne Femurteil). Die Prothese umfaßt eine metallische Trägerplattform 1, die in geeigneter Weise am Tibiaknochen implantiert wird. Die Trägerplattform 1 besitzt eine sich in Figur 2 nach oben erstreckende Lagerfläche 2, einen sich nach unten erstreckenden zentralen Stiel 3, der konisch ausgebildet ist und in dem eine konische Ausnehmung 4 angeordnet ist, sowie zwei sich von der Lagerfläche 2 nach oben erstreckende Seitenwände 5. Am unteren Ende des Stieles befindet sich ein Aussengewinde 13, mit dem Verlängerungsstiele verschraubt werden können.

In der Draufsicht hat die Trägerplattform 1 etwa die Form eines Halbmondes mit abgerundeten Ecken. An der in Figur 1 linken und rechten Außenseite (medial und lateral) befindet sich eine Seitenwand 5, die nach oben vorsteht sowie eine anteriore und posteriore Begrenzung aufweist, zwischen denen sich eine konkave Innenfläche erstreckt. Die Krümmung dieser Fläche besitzt einen konstanten Radius, so daß die Seitenwände letztendlich Ausschnitte aus einem Zylinder darstellen.

Die Lagerfläche 2 der Trägerplattform 1 ist eben ausgebildet, abgesehen von einer nach oben vorstehenden Nase 11, die zentral am Rand der Lagerfläche angeordnet ist und deren Funktion später beschrieben wird. Die im Stiel befindliche konische Ausnehmung 4 erweitert sich nach oben und geht am oberen Ende in einen erweiteren zylindrischen Bohrungsabschnitt 14 über.

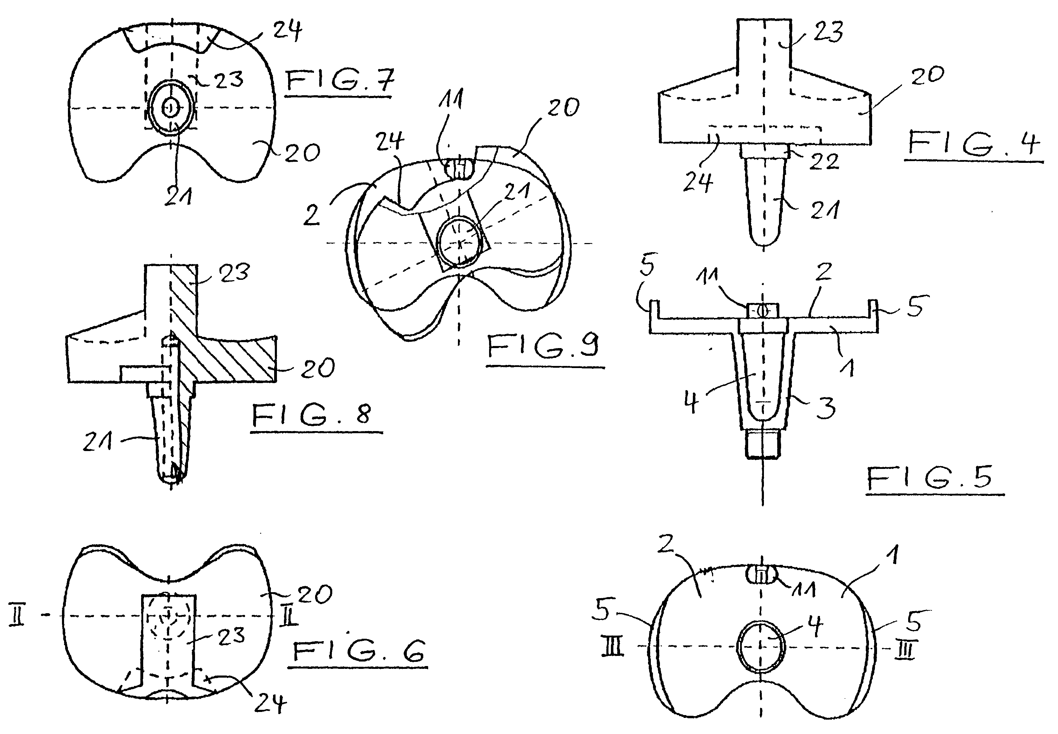

Auf der Trägerplattform 1 ist ein metallischer Aufsatz 6 zenral fixiert, d.h. drehfest angeordnet. Dieser Aufsatz ist in Figur 3 im einzelnen dargestellt und besitzt einen sich in Figur 2 nach unten erstreckenden Ansatz 15, der in die zylindrische Ausnehmung 14 der Trägerplattform eingepasst ist. Im übrigen liegt der Aufsatz 6, der insgesamt etwa Zylinderform besitzt, auf der Lagerfläche 2 der Trägerplattform auf.

Zur Fixierung besitzt der Aufsatz 6 an seinem Rand eine Ausnehmung 10, die mit der von der Trägerplattform nach oben vorstehenden Nase 11 zusammenwirkt. Durch diese Nase 11 erstreckt sich in Horizontalrichtung eine Fixierungsschraube, die in eine entsprechende Bohrung des Aufsatzes eingreift und mit dem Innengewinde dieser Bohrung verschraubt ist.

An seinem Aussenrand weist der Aufsatz 6 eine sich in Umfangsrichtung erstreckende Hinterschneidung 8 auf, die mit einem in Umfangsrichtung verlaufenden Vorsprung von zwei Polyethylen-Gleitlagern 9 zusammenwirkt. Die Gleitlager 9 besitzen die in den Figuren 1 und 2 gezeigte Form mit konkaver Oberfläche, auf der sich eine entsprechende Femurkomponente (nicht gezeigt) abstützt. Sie werden innen vom Rand des Aufsatzes 6 und aussen von der Innenfläche der Seitenwände 5 der Trägerplattform geführt. Beide Gleitlager 9 können sich somit entlang der zwischen den Seitenwänden und dem Aufsatz gebildeten Kreisringbahn in Anterior- und Posterior-Richtung (d.h. in Figur 1 nach oben und unten) bewegen. Da sie in die Hinterschneidung 8 des Aufsatzes greifen, ist eine Bewegung in Axialrichtung nicht möglich.

Die hier gezeigte Prothesenausführungsform findet dann Anwendung, wenn das hintere Kreuzband noch funktionsfähig ist. Im Falle einer Instabilität (ob nach mehreren Jahren post op oder direkt) kann der Zentralaufsatz 6 von der Trägerplattform 1 abgekoppelt werden, und die beweglichen Gleitlager 9 können entfernt werden. Stattdessen kann ein einstückiges Rotationsplateau, wie in den Figuren 4 bis 9 gezeigt, oder ein zweiteiliges Rotationsplateau der Figuren 10 und 11 installiert werden.

Es wird nunmehr zuerst auf die Ausführungsform mit dem einstückigen Rotationsplateau Bezug genommen. Dieses Plateau ist beispielsweise in Figur 4 dargestellt und mit 20 bezeichnet. Es wirkt mit der gleichen Trägerplattform 1 zusammen, die vorstehend in Verbindung mit den Figuren 1 bis 3 beschrieben wurde. Auf den Aufbau dieser Trägerplattform braucht daher an dieser Stelle im einzelnen nicht mehr eingegangen zu werden. Wesentlich ist, daß die von der Lagerfläche 2 der Trägerplattform 1 nach oben vorstehende Nase 11 bei dieser Ausführungsform eine Einrichtung zur Begrenzung der Rotation des Rotationsplateaus 20 bildet, die mit einer Ausnehmung 24 an der Unterseite des Rotationsplateaus 20 zusammenwirkt.

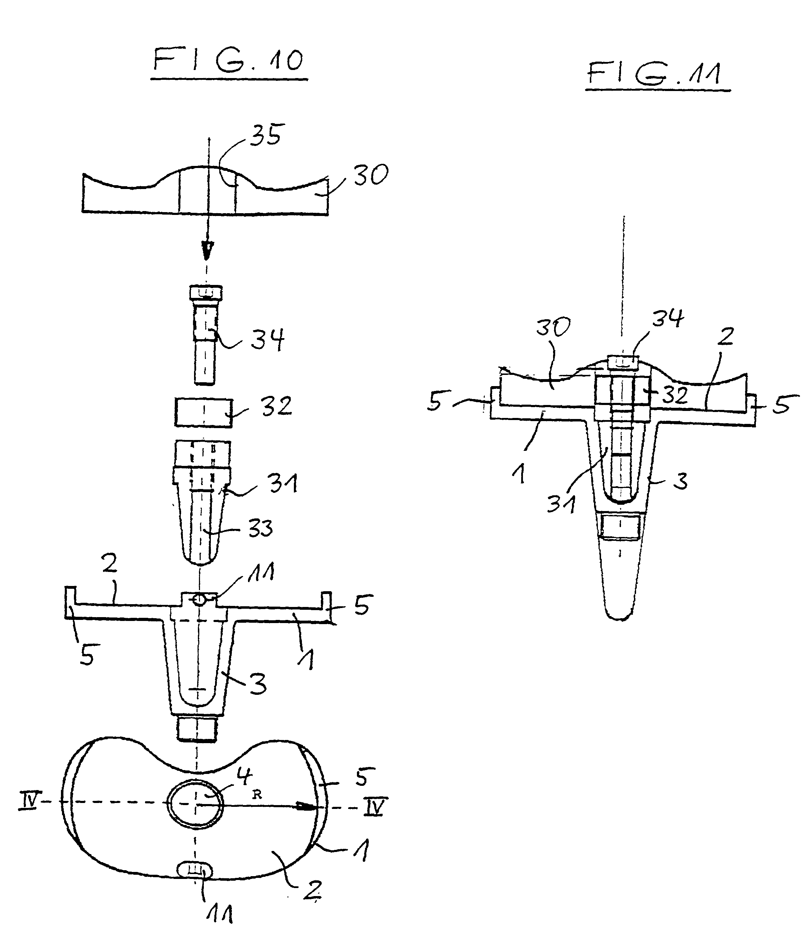

Das hier gezeigte Rotationsplateau 20 besitzt eine nach oben vorstehende zentrale Stabilisierungsnase 23 und zwei daneben angeordnete Lagerflächen für die Aufnahme und Lagerung eines speziellen Femurteils mit Stabilisierungskasten mit oder ohne Kopplung. Von der Unterseite des Rotationsplateaus 20 aus erstreckt sich ein im wesentlichen konischer Stiel 21 nach unten, dessen oberer Bereich 22 zylindrisch ausgebildet ist. Der Stiel passt somit in die aus den Abschnitten 14 und 4 gebildete Ausnehmung der Trägerplattform 1 und kann sich hierin drehen, wobei das eigentliche Plateau 20 auf der Lagerfläche der Trägerplattform gleitet. Die Drehung wird durch die erwähnte Nase 11 begrenzt, die mit dem Rand der Ausnehmung 24 an der Unterseite des Rotationsplateaus 20 zusammenwirkt. Wie in Figur 9 gezeigt, ist beispielsweise ein maximaler Rotationswinkel von 25 Grad vorgesehen.

Das ebenfalls etwa halbmondförmig ausgebildete Rotationsplateau 20 ist so breit, daß es an den Seitenwänden 5 der Trägerplattform 1 anliegt. Es wird somit bei seiner Drehbewegung sowohl von den Seitenflächen 5 als auch von der Seitenwand der zentralen Ausnehmung in der Trägerplattform geführt.

Die Figuren 10 und 11 zeigen ein zweiteiliges Rotationsplateau aus Polyethylen, das sich aus einem Plateauteil 30 und einem Drehzapfen 31 zusammensetzt. Das Plateauteil 30 besitzt eine zentrale Bohrung 35, die sich von der Oberseite zur Unterseite des Plateauteiles erstreckt. Der Drehzapfen 31 ist im wesentlichen konisch ausgebildet und besitzt eine Stahlhülse 32, die auf dem oberen Bereich des Zapfens 31 vormontiert ist. Bei der Installation des Rotationsplateaus auf der Trägerplattform 1, die im übrigen mit den vorstehend beschriebenen Trägerplattformen identisch ist, wird nach Entfernung des Aufsatzes und der Gleitlager das Plateauteil 30 von schräg anterior zwischen die Seitenwände 5 auf die Trägerplattform 1 aufgesetzt. Zur Stabilisierung wird nunmehr von oben der Drehzapfen 31 mit der vormontierten Metallhülse 32 in Richtung distal in die zentrale Ausnehmung 4 der Trägerplattform eingesetzt und mit dem Plateauteil 30 fest verbunden. Dies geschieht durch das Einschrauben einer Spannschraube 34, über die Zapfen 31 am Plateauteil 30 (durch Expansion) festgesetzt wird. Auch bei dieser Ausführungsform sorgt die Nase 11 der Trägerplattform, die mit einer entsprechenden Ausnehmung im Plateauteil 30 zusammenwirkt, für eine Rotationsbegrenzung desselben.

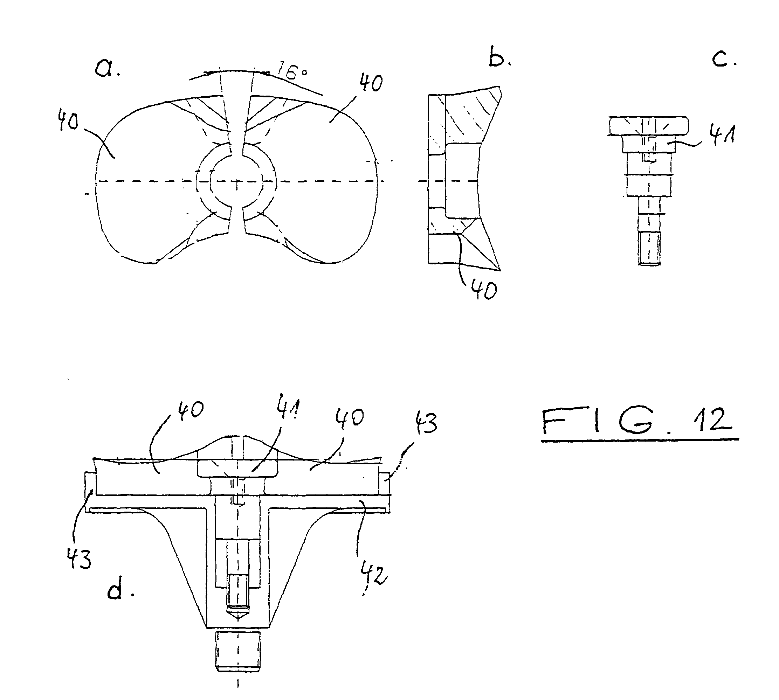

Die in Figur 12 gezeigte Ausführungsform einer Kniegelenk-Endoprothese besitzt eine zentrale Führung 41, die sowohl als Führung für zwei Gleitlager 40 als auch als Führung (Drehzapfen) für ein Rotationsplateau verwendbar ist. Bei dieser Ausführungsform sind die beiden Gleitlager 40 größer ausgebildet als bei der Ausführungsform der Figur 2. Sie werden auch hier wieder von der zentralen Führung 41 und den beiden Seitenwänden 43 der Trägerplattform 42 geführt. Die zentrale Führung 41 entspricht somit dem Aufsatz 6 bei der Ausführungsform der Figur 2.

Zur Vereinfachung ist diese zentrale Führung 41 so ausgebildet, daß sie auch für ein Rotationsplateau verwendbar ist, so daß Extrateile hierfür entfallen können. Die Führung 41 bildet dabei den Drehzapfen des Rotationsplateaus und wird hierzu in geeigneter Weise am Rotationsplateau (nicht gezeigt) fixiert.

## Patentansprüche

1. Kniegelenk-Endoprothesen-Bausatz, der die folgenden Bestandteile umfaßt:
(a) eine Trägerplattform (1) mit einer Lagerfläche (2) und einer nach oben vorstehenden medialen sowie einer nach oben vorstehenden lateralen Seitenwand (5), die sich gegenüberliegen und Teile einer Zylinderwand mit definiertem Radius (R) bilden, sowie einem sich nach unten erstreckenden zentralen Stiel (3), in dem eine zentrale Ausnehmung (4) angeordnet ist;
(b) einen Aufsatz (6) zur zentralen drehfesten Anordnung auf der Trägerplattform (1);
(c) zwei zur Lagerung einer Femurkomponente dienende Gleitlager (9) zur beweglichen Installation zwischen dem Aufsatz (6) und den Seitenwänden (5) der Trägerplattform (1); und
(d) ein zur Lagerung einer Femurkomponente dienendes Rotationsplateau (20, 30) zur zentralen drehbaren Installation auf der Trägerplattform (1) mit Hilfe eines in die Ausnehmung (4) der Trägerplattform eingreifenden Zapfens (21, 31) als Ersatz für den Aufsatz (6) und die Gleitlager (9).

2. Kniegelenk-Endoprothesen-Bausatz nach Anspruch 1, **dadurch gekennzeichnet, daß** die Seitenwände (5) der Trägerplattform (1) und die äußere seitliche Begrenzungsfläche des Aufsatzes (6) die gleiche Krümmung besitzen.

3. Kniegelenk-Endoprothesen-Bausatz nach Anspruch 1 oder 2, **dadurch gekennzeichnet, daß** die Seitenwände (5) der Trägerplattform (1) und die äußere seitliche Begrenzungsfläche des Rotationsplateaus (20, 30) die gleiche Krümmung besitzen.

4. Kniegelenk-Endoprothesen-Bausatz nach Anspruch 3, **dadurch gekennzeichnet, daß** das Rotationsplateau (20,30) an den Seitenwänden (5) der Trägerplattform (1) relativ hierzu drehbar anliegt.

5. Kniegelenk-Endoprothesen-Bausatz nach Anspruch 4, **dadurch gekennzeichnet, daß** das Rotationsplateau (20,30) sowohl an den Seitenwänden (5) der Trägerplattform (1) als auch mit seinem Zapfen (21,31) in der Ausnehmung (4) der Trägerplattform (1) geführt ist.

6. Kniegelenk-Endoprothesen-Bausatz nach einem der vorangehenden Ansprüche, **dadurch gekennzeichnet, daß** das Rotationsplateau (20) und der Zapfen (21) einstückig ausgebildet sind.

7. Kniegelenk-Endoprothesen-Bausatz nach einem der Ansprüche 1 bis 5, **dadurch gekennzeichnet, daß** Rotationsplateau (30) und Zapfen (31) zweiteilig ausgebildet und über Befestigungseinrichtungen aneinander befestigbar sind.

8. Kniegelenk-Endoprothesen-Bausatz nach einem der vorangehenden Ansprüche, **dadurch gekennzeichnet, daß** er eine Begrenzungseinrichtung für die Drehung des Rotationsplateaus (20, 30) aufweist.

9. Kniegelenk-Endoprothesen-Bausatz nach Anspruch 8, **dadurch gekennzeichnet, daß** die Begrenzungseinrichtung durch eine Nase (11) an der Trägerplattform (1) und eine hiermit zusammenwirkende Ausnehmung (24) am Rotationsplateau (20, 30) gebildet ist.

10. Kniegelenk-Endoprothesen-Bausatz nach Anspruch 9, **dadurch gekennzeichnet, daß** der Aufsatz (6) eine mit der Nase (11) der Trägerplattform (1) zusammenwirkende Ausnehmung (10) besitzt und über ein sich von der Nase (11) in den Aufsatz (6) erstreckendes Fixierelement an der Trägerplattform (1) fixiert ist.

11. Kniegelenk-Endoprothesen-Bausatz nach einem der vorangehenden Ansprüche, **dadurch gekennzeichnet, daß** das Rotationsplateau (20) eine nach oben ragende Stabilisierungsnase (23) zur Aufnahme eines Femurteiles aufweist.

12. Kniegelenk-Endoprothesen-Bausatz nach einem der vorangehenden Ansprüche, **dadurch gekennzeichnet, daß** am unteren Ende des Stieles (3) der Trägerplattform (1) Befestigungsmittel für Verlängerungsstiele vorgesehen sind.

13. Kniegelenk-Endoprothesen-Bausatz nach einem der vorangehenden Ansprüche, **dadurch gekennzeichnet, daß** die Trägerplattform (1) und der Aufsatz (6) aus Metall bestehen.

14. Kniegelenk-Endoprothesen-Bausatz nach einem der vorangehenden Ansprüche, **dadurch gekennzeichnet, daß** die Gleitlager (9) und das Rotationsplateau (20, 30) aus Kunststoff bestehen.

## Claims

1. An assembly set for knee prostheses comprising the following components:
(a) a support platform (1) including a support surface (2) and an upwardly projecting medial and an upwardly projecting lateral side-wall (5), said side-walls being disposed oppositely with respect to one another and forming parts of a cylinder wall with a defined radius (R), and an downwardly extending central post (3) in which a central recess (4) is disposed;
(b) a top (6) for a central pivotely fixed disposal on said support platform (1);
(c) two slide bearings (9) for the support of a femur component for the movable installation between said top (6) and the side-walls (5) of said support platform (1); and
(d) a rotary plateau (20, 30) serving for the support of a femur component for the central pivotal installation on said support platform (1) by means of a pivot (21, 31) engaging into the recess (4) of said support platform as substitute for said top (6) and said slide bearings (9).

2. The assembly set for knee prostheses according to claim 1, **characterized in that** the side-walls (5) of said support platform (1) and the outer lateral limiting surface of the top (6) have the same curvature.

3. The assembly set for knee prostheses according to claim 1 or 2, **characterized in that** the side-walls (5) of said support platform (1) and the outer lateral limiting surface of said rotary plateau (20, 30) have the same curvature.

4. The assembly set for knee prostheses according to claim 3, **characterized in that** said rotary plateau (20, 30) sits close to the side-walls (5) of said support platform (1) in a rotary manner with respect to the same.

5. The assembly set for knee prostheses according to claim 4, **characterized in that** said rotary plateau (20, 30) is guided not only at the side-walls (5) of said support platform (1) but also with its post (21, 31) in the recess (4) of said support platform (1).

6. The assembly set for knee prostheses according to one of the preceding claims, **characterized in that** said rotary plateau (20) and said post (21) are integrally formed.

7. The assembly set for knee prostheses according to one of the claims 1 to 5, **characterized in that** said rotary plateau (30) and said post (31) are formed as two parts and are fastened with respect to one another by fastening means.

8. The assembly set for knee prostheses according to one of the preceding claims, **characterized in that** it includes a limiting means for the rotation of the rotary plateau (20, 30).

9. The assembly set for knee prostheses according to claim 8, **characterized in that** said limiting means is formed by a nose (11) at said support platform (1) and a recess (24) at said rotary plateau (20, 30) for operating herewith.

10. The assembly set for knee prostheses according to claim 9, **characterized in that** said top (6) has a recess (10) cooperating with said nose (11) of said support platform (1) and is fixed at said support platform (1) by means of a fastening member extending from said nose (11) into said top (6).

11. The assembly set for knee prostheses according to one of the preceding claims, **characterized in that** said rotary plateau (20) has an upwardly projecting stabilizing nose (23) for the receipt of a femur part.

12. The assembly set for knee prostheses according to one of the preceding claims, **characterized in that** fastening means for extension posts are provided at the lower end of said post (3) of said support platform (1).

13. The assembly set for knee prostheses according to one of the preceding claims, **characterized in that** said support platform (1) and said top (6) consist of metal.

14. The assembly set for knee prostheses according to one of the preceding claims, **characterized in that** said slide bearings (9) and said rotary plateau (20, 30) consist of plastics.

## Revendications

1. Ensemble d'endoprothèse de l'articulation du genou, qui comprend les composants suivants :
(a) une plate-forme de support (1) dotée d'une surface de montage (2), d'une paroi médiale qui déborde vers le haut et d'une paroi latérale (5) qui déborde vers le haut, ces parois étant situées l'une en face de l'autre et formant des parties d'une paroi cylindrique de rayon (R) défini, ainsi qu'un montant central (3) qui s'étend vers le bas et dans lequel est disposée une découpe centrale (4),
(b) un gradin (6) destiné à être placé au centre de la plate-forme de support (1) sans pouvoir y tourner,
(c) deux paliers coulissants (9) qui servent à monter un composant de fémur de manière à l'installer à déplacement entre le gradin (6) et les parois latérales (5) de la plate-forme de support (1) et
(d) un plateau de rotation (20, 30) qui sert à monter un composant de fémur de manière à l'installer à rotation sur la plate-forme de support (1) à l'aide d'un tourillon (21, 31) qui s'engage dans la découpe (4) de la plate-forme de support (1) pour remplacer le gradin (6) et les paliers coulissants (9).

2. Ensemble d'endoprothèse de l'articulation du genou selon la revendication 1, **caractérisé en ce que** les parois latérales (5) de la plate-forme de support (1 ) et la surface latérale extérieure qui délimite le gradin (6) ont la même courbure.

3. Ensemble d'endoprothèse de l'articulation du genou selon la revendication 1 ou la revendication 2, **caractérisé en ce que** les parois latérales (5) de la plate-forme de support (1) et la surface latérale extérieure qui délimite le plateau de rotation (20, 30) ont la même courbure.

4. Ensemble d'endoprothèse de l'articulation du genou selon la revendication 3, **caractérisé en ce que** le plateau de rotation (20, 30) repose contre les parois latérales (5) de la plate-forme de support (1) à rotation par rapport à ces dernières.

5. Ensemble d'endoprothèse de l'articulation du genou selon la revendication 4, **caractérisé en ce que** le plateau de rotation (20, 30) est guidé dans la découpe (4) de la plate-forme de support (1) tant sur les parois latérales (5) de la plate-forme de support (1) que par son tourillon (21, 31).

6. Ensemble d'endoprothèse de l'articulation du genou selon l'une des revendications précédentes, **caractérisé en ce que** le plateau de rotation (20) et le tourillon (21) sont réalisés en une seule pièce.

7. Ensemble d'endoprothèse de l'articulation du genou selon l'une des revendications 1 à 5, **caractérisé en ce que** le plateau de rotation (30) et le tourillon (31) sont réalisés en deux pièces et peuvent être fixés l'un contre l'autre par des dispositifs de fixation.

8. Ensemble d'endoprothèse de l'articulation du genou selon l'une des revendications précédentes, **caractérisé en ce qu'**il présente un dispositif qui limite la rotation du plateau de rotation (20, 30).

9. Ensemble d'endoprothèse de l'articulation du genou selon la revendication 8, **caractérisé en ce que** le dispositif qui limite la rotation est formé par un bec (11) prévu sur la plate-forme de support (1) et une découpe (24) ménagée sur le plateau de rotation (20, 30) et qui coopère avec le bec.

10. Ensemble d'endoprothèse de l'articulation du genou selon la revendication 9, **caractérisé en ce que** le gradin (6) possède une découpe (10) qui coopère avec le bec (11) prévu sur la plate-forme de support (1) et est fixé sur la plate-forme de support (1) par un élément de fixation qui part du bec (11) et qui s'étend jusque dans le gradin (6).

11. Ensemble d'endoprothèse de l'articulation du genou selon l'une des revendications précédentes, **caractérisé en ce que** le plateau de rotation (20) présente un bec de stabilisation (23) qui déborde vers le haut et qui sert à recevoir une partie de fémur.

12. Ensemble d'endoprothèse de l'articulation du genou selon l'une des revendications précédentes, **caractérisé en ce que** des moyens de fixation de montants de prolongation sont prévus à l'extrémité inférieure du montant (3) de la plate-forme de support (1).

13. Ensemble d'endoprothèse de l'articulation du genou selon l'une des revendications précédentes, **caractérisé en ce que** la plate-forme de support (1) et le gradin (6) sont réalisés en métal.

14. Ensemble d'endoprothèse de l'articulation du genou selon l'une des revendications précédentes, **caractérisé en ce que** les paliers coulissants (9) et le plateau de rotation (20, 30) sont réalisés en matière synthétique.
